# EUROPEAN PATENT SPECIFICATION

(11) **EP 2 996 719 B1**
(45) Date of publication and mention of the grant of the patent: **26.06.2019**
(21) Application number: 14729191.8
(22) Date of filing: 12.05.2014
(51) Int. Cl.: A61K 39/39, A61K 39/008, A61K 39/27, A61K 39/00, A61K 39/12

(54) **NOVEL VACCINE COMPOSITIONS COMPRISING IMMUNOSTIMULATORY OLIGONUCLEOTIDES**
NEUARTIGE IMPFSTOFFZUSAMMENSETZUNGEN MIT IMMUNSTIMULIERENDEN OLIGONUKLEOTIDEN
NOUVELLES COMPOSITIONS DE VACCIN COMPRENANT DES OLIGONUCLÉOTIDES IMMUNOSTIMULATEURS

(30) Priority: 14.05.2013 US 201361823189 P
(43) Date of publication of application: 23.03.2016
(60) Divisional application: 19173426.8
(73) Proprietor: Zoetis Services LLC, Parsippany, NJ 07054 (US)
(72) Inventor: DOMINOWSKI, Paul, Joseph, Kalamazoo, MI 49007 (US); RAI, Sharath, K., Kalamazoo, MI 49007 (US); SLY, Laurel, Mary, Kalamazoo, MI 49007 (US); COOK, Corey, Patrick, Kalamazoo, MI 49007 (US); MWANGI, Duncan, Kalamazoo, MI 49007 (US); FOSS, Dennis, L., Kalamazoo, MI 49007 (US); KREBS, Richard, Lee, Lincoln, NE 68521 (US)
(74) Representative: Mannion, Sally Kim
(86) International application number: PCT/US2014/037705
(87) International publication number: WO 2014/186291

(56) References cited:
- WO-A1-2011/148356
- WO-A2-2008/068638
- CHRISTIAN BODE ET AL: "CpG DNA as a vaccine adjuvant", EXPERT REVIEW OF VACCINES, vol. 10, no. 4, 1 April 2011 (2011-04-01), pages 499-511, XP055138146, ISSN: 1476-0584, DOI: 10.1586/erv.10.174
- STACEY K J ET AL: "Immunostimulatory DNA as an adjuvant in vaccination against Leishmania major", INFECTION AND IMMUNITY, AMERICAN SOCIETY FOR MICROBIOLOGY, US, vol. 67, no. 8, 1 August 1999 (1999-08-01) , pages 3719-3726, XP002977881, ISSN: 0019-9567
- OLIVEIRA-FREITAS ET AL: "Acylated and deacylated saponins of Quillaja saponaria mixture as adjuvants for the FML-vaccine against visceral leishmaniasis", VACCINE, ELSEVIER, AMSTERDAM, NL, vol. 24, no. 18, 1 May 2006 (2006-05-01), pages 3909-3920, XP005421532, ISSN: 0264-410X, DOI: 10.1016/J.VACCINE.2006.02.034

### SEQUENCE LISTING

<110> ZOETIS LLC DOMINOWSKI, PAUL JOSEPH RAI, SHARATH K. SLY, LAUREL MARY COOK, COREY PATRICK MWANGI, DUNCAN FOSS, DENNIS L. KREBS, RICHARD LEE
<120> NOVEL VACCINE COMPOSITIONS COMPRISING IMMUNOSTIMULATORY OLIGONUCLEOTIDES
<130> ZP000011A
<150> US 61/823189
   <151> 2013-05-14
<160> 10
<170> PatentIn version 3.5
<210> 1
   <211> 23
   <212> DNA
   <213> Artificial Sequence
<220>
   <223> CpG oligonucleotide
<400> 1
   tcgtcgacga tcggcgcgcg ccg 23
<210> 2
   <211> 23
   <212> DNA
   <213> Artificial Sequence
<220>
   <223> CpG oligonucleotide
<400> 2
   tcgacgtcga tcggcgcgcg ccg 23
<210> 3
   <211> 24
   <212> DNA
   <213> Artificial Sequence
<220>
   <223> CpG oligonucleotide
<400> 3
   tcgacgtcga tcggcgcgcg ccgt 24
<210> 4
   <211> 23
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 4
   tcgacgtcga tcggcgcgcg ccg 23
<210> 5
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 5
   tcgacgtcga tcggcgcgcg ccgt 24
<210> 6
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 6
   tcgacgtcga tcggcgcgcg ccgt 24
<210> 7
   <211> 23
   <212> DNA
   <213> Artificial Sequence
<220>
   <223> CpG oligonucleotide
<220>
   <221> modified_base
   <222> (1)..(1)
   <223> 5'-Ethyl-2'-deoxyuridine
<400> 7
   tcgacgtcga tcggcgcgcg ccg 23
<210> 8
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 8
   tcgtcgacga tcggcggccg ccgt 24
<210> 9
   <211> 24
   <212> DNA
   <213> Artificial Sequence
<220>
   <223> CpG oligonucleotide
<220>
   <221> modified_base
   <222> (1)..(1)
   <223> 5'-Iodo-2'-deoxyuridine
<400> 9
   tcgtcgacga tcggcggccg ccgt 24
<210> 10
   <211> 23
   <212> DNA
   <213> Artificial Sequence
<220>
   <223> CpG oligonucleotide
<400> 10
   tcgtcgacga tcggcgcgcg ccg 23

## Description

### FIELD OF THE INVENTION

This invention relates to novel adjuvants for enhancing immune response to Leishmania vaccines.

### BACKGROUND

Leishmaniasis is a major and severe parasitic disease of humans, canids (dogs, wolves, foxes, coyotes, jackals), and felids (lions, tigers, domestic cats, wild cats, other big cats, and other felines including cheetahs and lynx). The agent of leishmaniasis is a protozoan parasite and belongs to the leishmania donovani complex. This parasite is widely distributed in temperate and subtropical countries of Southern Europe, Africa, Asia, South America and Central America (Desjeux P., Trans. R. Soc. Trop. Med. Hyg., 2001, 95: 239-43). Leishmania donovani infantum (L. infantum) is responsible for the feline and canine disease in Southern Europe, Africa, and Asia. In South America and Central America, the agent is Leishmania donovani chagasi (L. chagasi), which is closely related to L. infantum. In humans, the agent is Leishmania donovani donovani (L. donovani), which is closely related to L. infantum and L. chagasi.

These parasites cause visceral leishmaniasis and/or cutaneous leishmaniasis. Visceral leishmaniasis results in clinical symptoms like fever, cachexia, hepatosplenomegaly (enlargement of the liver and spleen), and blood cytopenia. Cutaneous leishmaniasis occurs in varying presentations, from the self-limited and even self-healing cutaneous forms to fatal systemic disease. Lesions of cutaneous leishmaniasis may occur anywhere on the body but the most common sites are those which are exposed to the environment and are therefore more susceptible to bites from the sand flies. The initial papule rapidly gives rise to an ulcer. Systemic leishmaniasis is rare but is invariably fatal if not treated promptly. Systemic leishmaniasis affects the internal body organs, specifically the spleen and the liver.

In canines, the disease is associated with cutaneous symptoms or with visceral symptoms or both cutaneous and visceral symptoms, and is lethal in the absence of therapy.

Numerous treatments have been described but none is fully satisfactory due to toxicity of the treatment itself or a tendency for the animal to relapse.

Bode et al (Expert Review of Vaccines 2011; 10(4) 499-511) discusses different types of CpG oligonucleotides and their potential as adjuvants in general.

Davis et al (WO2011/148356) discloses a combination of CpG oligonucleotides and cholesterol.

Debelak et al (WO2008/068638) discloses P-class CpG oligonucleotides having 5' base substituted with lipophilic analogs. Stacey et al (Infection and Immunity 1999, 67(8): 3719-3726) discusses usefulness of immunostimulatory DNA as an adjuvant in an anti-Leishmania vaccine. Olivera-Freitas et al., (Vaccine, 2006, 24(18): 3909-3920) discloses Leishmania FML vaccine comprising saponins.

Accordingly, new vaccine formulations are needed to protect domestic animals against Leishmania infections.

### SUMMARY OF INVENTION

The instant invention addresses drawbacks of the art by providing, in one aspect, a vaccine composition comprising an antigen component wherein the antigen component is a Leishmania antigen, and an adjuvant component, wherein the adjuvant component comprises a P-class immunostimulatory oligonucleotide and a combination of a saponin and a sterol.

Preferably, the P-class immunostimulatory oligonucleotide, which may be modified and/or stabilized, is present in the amount of 20-500 µg per dose, e.g., 100-250 µg per dose.

Preferably, the adjuvant component comprises a saponin, such as Quil A or a purified fraction thereof, and a sterol, such as cholesterol, each in the amount of between about 5-100 µg/ml. In a specific embodiment, each of Quil A and cholesterol is present in the amount of 50 µg/ml.

In certain embodiments of the invention, the vaccine formulation is non-liposomal.

### DETAILED DESCRIPTION OF SELECTED EMBODIMENTS

The following definitions are provided for a better understanding of the invention:

"About" or "approximately," when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater, unless about is used in reference to time intervals in weeks where "about 3 weeks," is 17 to 25 days, and about 2 to about 4 weeks is 10 to 40 days.

"Adjuvant" means any substance that increases the humoral or cellular immune response to an antigen. Adjuvants are generally used to accomplish two objectives: The slow the release of antigens from the injection site, and the stimulation of the immune system.

"Antibody" refers to an immunoglobulin molecule that can bind to a specific antigen as the result of an immune response to that antigen. Immunoglobulins are serum proteins composed of "light" and "heavy" polypeptide chains having "constant" and "variable" regions and are divided into classes (e.g., IgA, IgD, IgE, IgG, and IgM) based on the composition of the constant regions.

"Antigen" or "immunogen" refers to any substance that stimulates an immune response. The term includes killed, inactivated, attenuated, or modified live bacteria, viruses, or parasites. The term antigen also includes polynucleotides, polypeptides, recombinant proteins, synthetic peptides, protein extract, cells (including tumor cells), tissues, polysaccharides, or lipids, or fragments thereof, individually or in any combination thereof. The term antigen also includes antibodies, such as anti-idiotype antibodies or fragments thereof, and to synthetic peptide mimotopes that can mimic an antigen or antigenic determinant (epitope).

"Bacterin" means a suspension of one or more killed bacteria which may be used as a component of a vaccine or immunogenic composition.

"Buffer" means a chemical system that prevents change in the concentration of another chemical substance, e.g., proton donor and acceptor systems serve as buffers preventing marked changes in hydrogen ion concentration (pH). A further example of a buffer is a solution containing a mixture of a weak acid and its salt (conjugate base) or a weak base and its salt (conjugate acid).

"Cellular immune response" or "cell mediated immune response" is one mediated by T-lymphocytes or other white blood cells or both, and includes the production of cytokines, chemokines and similar molecules produced by activated T-cells, white blood cells, or both.

"Consisting essentially" as applied to the adjuvant formulations refers to formulation which does not contain unrecited additional adjuvanting or immunomodulating agents in the amounts at which said agent exert measurable adjuvanting or immunomodulating effects.

"Dose" refers to a vaccine or immunogenic composition given to a subject. A "first dose" or "priming vaccine" refers to the dose of such a composition given on Day 0. A "second dose" or a "third dose" or an "annual dose" refers to an amount of such composition given subsequent to the first dose, which may or may not be the same vaccine or immunogenic composition as the first dose.

"Humoral immune response" refers to one that is mediated by antibodies.

"Immune response" in a subject refers to the development of a humoral immune response, a cellular immune response, or a humoral and a cellular immune response to an antigen. Immune responses can usually be determined using standard immunoassays and neutralization assays, which are known in the art.

"Immunologically protective amount" or "immunologically effective amount" or "effective amount to produce an immune response" of an antigen is an amount effective to induce an immunogenic response in the recipient. The immunogenic response may be sufficient for diagnostic purposes or other testing, or may be adequate to prevent signs or symptoms of disease, including adverse health effects or complications thereof, caused by infection with a disease agent. Either humoral immunity or cell-mediated immunity or both may be induced. The immunogenic response of an animal to an immunogenic composition may be evaluated, e.g., indirectly through measurement of antibody titers, lymphocyte proliferation assays, or directly through monitoring signs and symptoms after challenge with wild type strain, whereas the protective immunity conferred by a vaccine can be evaluated by measuring, e.g., reduction in clinical signs such as mortality, morbidity, temperature number, overall physical condition, and overall health and performance of the subject. The immune response may comprise, without limitation, induction of cellular and/or humoral immunity.

"Immunogenic" means evoking an immune or antigenic response. Thus an immunogenic composition would be any composition that induces an immune response.

"Immunostimulatory molecule" refers to a molecule that generates an immune response.

"Lipids" refers to any of a group of organic compounds, including the fats, oils, waxes, sterols, and triglycerides that are insoluble in water but soluble in nonpolar organic solvents, are oily to the touch, and together with carbohydrates and proteins constitute the principal structural material of living cells.

"Pharmaceutically acceptable" refers to substances, which are within the scope of sound medical judgment, suitable for use in contact with the tissues of subjects without undue toxicity, irritation, allergic response, and the like, commensurate with a reasonable benefit-to-risk ratio, and effective for their intended use.

"Reactogenicity" refers to the side effects elicited in a subject in response to the administration of an adjuvant, an immunogenic, or a vaccine composition. It can occur at the site of administration, and is usually assessed in terms of the development of a number of symptoms. These symptoms can include inflammation, redness, and abscess. It is also assessed in terms of occurrence, duration, and severity. A "low" reaction would, for example, involve swelling that is only detectable by palpitation and not by the eye, or would be of short duration. A more severe reaction would be, for example, one that is visible to the eye or is of longer duration.

"Room Temperature" means a temperature from 18 to 25°C.

"Saponin" refers to a group of surface-active glycosides of plant origin composed of a hydrophilic region (usually several sugar chains) in association with a hydrophobic region of either steroid or triterpenoid structure.

"Steroids" refers to any of a group of organic compounds belonging to biochemical class of lipids, which are easily soluble in organic solvents and slightly soluble in water. Steroids comprise a four-fused ring system of three fused cyclohexane (six-carbon) rings plus a fourth cyclopentane (five-carbon) ring.

"Sterols" refers to compounds in animals which are biologically produced from terpenoid precursors. They comprise a steroid ring structure, having a hydroxyl (OH) group, usually attached to carbon-3. The hydrocarbon chain of the fatty-acid substituent varies in length, usually from 16 to 20 carbon atoms, and can be saturated or unsaturated. Sterols commonly contain one or more double bonds in the ring structure and also a variety of substituents attached to the rings. Sterols and their fatty-acid esters are essentially water insoluble.

"Subject" refers to any animal for which the administration of an adjuvant composition is desired. It includes mammals and non-mammals, including primates, livestock, companion animals, laboratory test animals, captive wild animals, aves (including in ova), reptiles, and fish. Thus, this term includes but is not limited to monkeys, humans, swine; cattle, sheep, goats, equines, mice, rats, guinea pigs, hamsters, rabbits, felines, canines, chickens, turkeys, ducks, other poultry, frogs, and lizards.

"Therapeutically effective amount" refers to an amount of an antigen or vaccine that would induce an immune response in a subject receiving the antigen or vaccine which is adequate to prevent or reduce signs or symptoms of disease, including adverse health effects or complications thereof, caused by infection with a pathogen, such as a virus or a bacterium. Humoral immunity or cell-mediated immunity or both humoral and cell-mediated immunity may be induced. The immunogenic response of an animal to a vaccine may be evaluated, e.g., indirectly through measurement of antibody titers, lymphocyte proliferation assays, or directly through monitoring signs and symptoms after challenge with wild type strain. The protective immunity conferred by a vaccine can be evaluated by measuring, e.g., reduction in clinical signs such as mortality, morbidity, temperature number, overall physical condition, and overall health and performance of the subject. The amount of a vaccine that is therapeutically effective may vary depending on the particular adjuvant used, the particular antigen used, or the condition of the subject, and can be determined by one skilled in the art.

"Treating" refers to preventing a disorder, condition, or disease to which such term applies, or to preventing or reducing one or more symptoms of such disorder, condition, or disease.

"Treatment" refers to the act of "treating" as defined above.

"Triterpeniods" refers to a large and diverse class of naturally occurring organic molecules, derived from six five-carbon isoprene (2-methyl-1,3-butadiene) units, which can be assembled and modified in thousands of ways. Most are multicyclic structures which differ from one another in functional groups and in their basic carbon skeletons. These molecules can be found in all classes of living things.

"Vaccine" refers to a composition that includes an antigen, as defined herein. Administration of the vaccine to a subject results in an immune response, generally against one or more specific diseases. The amount of a vaccine that is therapeutically effective may vary depending on the particular antigen used, or the condition of the subject, and can be determined by one skilled in the art.

As noted above, the instant invention provides a vaccine composition comprising an antigen component which is a Leishmania antigen, and an adjuvant component, wherein the adjuvant component comprises (or consists essentially of, or consists of) a P-class immunostimulatory oligonucleotide and a combination of a saponin and a sterol.

Where the oil is present, the antigen would generally be dispersed or dissolved in the aqueous phase and not enveloped by liposomes or similar structures. In vaccine compositions where the saponin and the sterol are present, said saponins and said sterols would form helical micelles and the antigen would be in admixture with, but not integrated within, said helical micelles.

### Antigens

Different antigens derived from Leishmania are suitable for the instant invention. The antigens may include, without limitation, whole organisms (inactivated, attenuated, and modified live), nucleotides, polynucleotides, peptides, polypeptides, recombinant proteins, synthetic peptides, protein extract, polysaccharides, carbohydrates, fatty acids, teichioc acid, peptidoglycans, lipids, or glycolipids, individually or in any combination thereof.

The amount of antigen used to induce an immune response will vary considerably depending on the antigen used, the subject, and the level of response desired, and can be determined by one skilled in the art.

The number of cells for a Leishmania antigen administered in a vaccine ranges from about 1x10² to about 1x10¹⁰ per dose, inclusive. In other embodiments, the number of cells ranges from about 1x 10³ to about 1x10⁹ per dose, inclusive, or from about 1x10⁴ to about 1x10⁸ per dose, inclusive, or from about 1x10⁵ to about 1x10⁷ per dose, inclusive, or from about 1x10⁶ to about 1x10⁸ per dose, inclusive.

A therapeutically effective amount of antigen in vaccines containing inactivated viruses can also be measured in terms of Relative Potency (RP) per mL. A therapeutically effective amount is often in the range from about 0.1 to about 50 RP per mL, inclusive. In other embodiments, the therapeutically effective amount of the antigen is in a range from about 0.5 to about 30 RP per mL, inclusive, from about 1 to about 25 RP per mL, inclusive, from about 2 to about 20 RP per mL, inclusive, from about 3 to about 15 RP per mL, inclusive, or from about 5 to about 10 RP per mL, inclusive.

Suitable examples of Leishmania antigens include whole organisms, protein extracts, glycoproteins, outer capsid proteins and extracts, and nucleic acid sequences which encode said outer capsid proteins. Specific antigens may include Gp63, PSA-2 and Fucose Mannose Ligand. In another embodiment, leishmanial excretory-secretory antigens (ESAs) may be used. ESAs for at least of seventeen ESAs of relative molecular weights 11, 13, 16, 18, 21, 23, 26, 29, 33, 35, 42, 51, 54, 58, 64, 70 and 80 kDa have been identified. Two fractions, F1 (11, 13 and 16 kDa) and F3 (26, 29 and 33 kDa), were found to be highly immunogenic as they significantly induced NADPH oxidase and SOD activities as well as NOx, TNF-α, IFN-γ and IL-12 production in stimulated RAW 264.7 macrophages. Further, these antigens also induced significant proliferation of human peripheral blood mononuclear cells along with increased production of IFN-γ and IL-12. See Gour et al., Experimental Parasitology, 2012; 132(3), 355-361.

### Saponins and CpGs

Triterpenoid saponins suitable for use in the adjuvant compositions can come from many sources, either plant derived or synthetic equivalents, including but not limited to, Quillaja saponaria, tomatine, ginsing extracts, mushrooms, and an alkaloid glycoside structurally similar to steroidal saponins. Thus, triterpenoids suitable for use in the adjuvant compositions include saponins, squalene, and lanosterol. The amount of triterpenoids suitable for use in the adjuvant compositions depends upon the nature of the triterpenoid used. However, they are generally used in an amount of about 1 µg to about 5,000 µg per dose. They also are used in an amount of about 1 µg to about 4,000 µg per dose, about 1 µg to about 3,000 µg per dose, about 1 µg to about 2,000 µg per dose, and about 1 µg to about 1,000 µg per dose. They are also used in an amount of about 5 µg to about 750 µg per dose, about 5 µg to about 500 µg per dose, about 5 µg to about 200 µg per dose, about 5 µg to about 100 µg per dose, about 15 µg to about 100 µg per dose, and in an amount of about 30 µg to about 75 µg per dose.

If a saponin is used, the adjuvant compositions generally contain an immunologically active saponin fraction from the bark of Quillaja saponaria. The saponin may be, for example, Quil A or another purified or partially purified saponin preparation, which can be obtained commercially. For example, Quil A is sold in the USA by E.M Sergeant Company. QS-7, QS-17, QS-18, and QS-21 may be obtained from Antigenics Company, Massachusetts, USA. Thus, saponin extracts can be used as mixtures or purified individual components such as QS-7, QS-17, QS-18, and QS-21. In one embodiment the Quil A is at least 85% pure. In other embodiments, the Quil A is at least 90%, 91%, 92%, 93%, 94%, 95%, 96%, 97%, 98%, or 99% pure.

CpG oligonucleotides are a recently described class of pharmacotherapeutic agents that are characterized by the presence of an unmethylated CG dinucleotide in specific base-sequence contexts (CpG motif). (Hansel TT, Barnes PJ (eds): New Drugs for Asthma, Allergy and COPD. Prog Respir Res. Basel, Karger, 2001, vol 31, pp 229-232). These CpG motifs are not seen in eukaryotic DNA, in which CG dinucleotides are suppressed and, when present, usually methylated, but are present in bacterial DNA to which they confer immunostimulatory properties.

The adjuvants of the instant invention utilize a so-called P-class immunostimulatory oligonucleotide, more preferably, modified P-class immunostimulatory oligonucleotides. P-class immunostimulatory oligonucleotides are CpG oligonucleotides characterized by the presence of palindromes, generally 6-20 nucleotides long. The P-Class oligonucleotides have the ability to spontaneously self-assemble into concatamers either in vitro and/or in vivo. These oligonucleotides are, in a strict sense, single-stranded, but the presence of palindromes allows for formation of concatamers or possibly stem-and-loop structures. The overall length of P-class immunostimulatory oligonucleotides is between 19 and 100 nucleotides, e.g., 19-30 nucleotides, 30-40 nucleotides, 40-50 nucleotides, 50-60 nucleotides, 60-70 nucleotides, 70-80 nucleotides, 80-90 nucleotides, 90-100 nucleotides.

In one aspect of the invention the immunostimulatory oligonucleotide contains a 5' TLR activation domain and at least two palindromic regions, one palindromic region being a 5' palindromic region of at least 6 nucleotides in length and connected to a 3' palindromic region of at least 8 nucleotides in length either directly or through a spacer.

The P-class immunostimulatory oligonucleotides may be modified according to techniques known in the art. For example, J-modification refers to iodo-modified nucleotides. E-modification refers to ethyl-modified nucleotide(s). Thus, E-modified P-class immunostimulatory oligonucleotides are P-class immunostimulatory oligonucleotides, wherein at least one nucleotide (preferably 5' nucleotide) is ethylated. Additional modifications include attachment of 6-nitro-benzimidazol, O-Methylation, modification with proynyl-dU, inosine modification, 2-bromovinyl attachment (preferably to uridine).

The P-class immunostimulatory oligonucleotides may also contain a modified internucleotide linkage including, without limitations, phosphodiester linkages and phosphorothioate linkages. The oligonucleotides of the instant invention may be synthesized or obtained from commercial sources.

P-Class oligonucleotides and modified P-class oligonucleotides are further disclosed in published PCT application no. WO2008/068638, published on Jun. 12, 2008. Suitable examples of modified P-class immunostimulatory oligonucleotides are provided below ("*" refers to a phosphorothioate bond and "_" refers to a phosphodiester bond).

| | |
|---|---|
| SEQ ID NO: 1 | 5' T*C-G*T*C-G*A*C-G*A*T*C-G*G*C*G*C-G*C*G*C*C*G 3' |
| SEQ ID NO: 2 | 5' T*C-G*A*C*G*T*C*G*A*T*C*G*G*C*G*C*G*C*G*C*C*G 3' |
| SEQ ID NO: 3 | 5' T*C*G*A*C*G*T*C*G*A*T*C*G*G*C*G*C*G*C*G*C*C*G*T 3' |
| SEQ ID NO: 4 | 5' JU*C-G*A*C*G*T*C*G*A*T*C*G*G*C*G*C*G*C*G*C*C*G 3' |
| SEQ ID NO: 5 | 5' JU*C-G*A*C*G*T*C*G*A*T*C*G*G*C*G*C*G*C*G*C*C* G*T 3' |
| SEQ ID NO: 6 | 5' JU*C*G*A*C*G*T*C*G*A*T*C*G*G*C*G*C*G*C*G*C*C* G*T 3' |
| SEQ ID NO: 7 | 5' EU*C-G*A*C*G*T*C*G*A*T*C*G*G*C*G*C*G*C*G*C*C*G 3' |
| SEQ ID NO: 8 | 5' JU*C-G*T*C*G*A*C*G*A*T*C*G*G*C*G*G*C*C*G*C*C* G*T 3' |
| SEQ ID NO: 9 | 5' JU*C*G*T*C*G*A*C*G*A*T*C*G*G*C*G*G*C*C*G*C*C* G*T 3' |
| SEQ ID NO: 10 | 5' T*C_G*T*C_G*A*C_G*A*T*C_G*G*C*G*C_G*C*G*C*C*G 3'. |

The amount of P-class immunostimulatory oligonucleotide for use in the adjuvant compositions depends upon the nature of the P-class immunostimulatory oligonucleotide used and the intended species. However, they are generally used in an amount of about 20 µg to about 500 µg per ml. They also are used in an amount of about 25 µg to about 400 mg per ml, about 40 µg to about 250 µg per ml, about 50 µg to about 200 µg per ml, about 100 g per ml to about 200 µg per ml.

### Sterols

Sterols suitable for use in the adjuvant compositions include β-sitosterol, stigmasterol, ergosterol, ergocalciferol, and cholesterol. These sterols are well known in the art and can be purchased commercially. For example cholesterol is disclosed in the Merck Index, 12th Ed., p. 369. The amount of sterols suitable for use in the adjuvant compositions depends upon the nature of the sterol used. However, they are generally used in an amount of about 1 µg to about 5,000 µg per ml. They also are used in an amount of about 1 µg to about 4,000 µg per ml, about 1 µg to about 3,000 µg per ml, about 1 µg to about 2,000 µg per ml, and about 1 µg to about 1,000 µg per ml. They are also used in an amount of about 5 µg to about 750 µg per ml, about 5 µg to about 500 µg per ml, about 5 µg to about 200 µg per ml, about 5 µg to about 100 µg per ml, about 15 µg to about 100 µg per ml, and about 30 µg to about 75 µg per ml.

The preparation of the compositions containing the saponin and the sterol according to the instant invention is within the ordinary skill in the art. Briefly, an aqueous mixture is prepared, said mixture comprising the antigen, the P-class immunomodulatory oligonucleotide, and the saponin. The sterol is then gradually (or dropwise) added to that mixture.

### Oily Phase

The adjuvant component may comprise an oily phase in the amount of about 2 to about 20% v/v of the vaccine composition, e.g., about 3 to about 15%, about 5 to about 15%, about 10 to about 15%, about 10 to about 20 % etc. The oily phase generally comprises an oil and, optionally, one or more emulsifiers. The oily phase may comprise two emulsifiers, one of which is lipid-soluble and the other is water-soluble.

Multiple oils and combinations thereof are suitable for use as disclosed herein. These oils include, without limitations, animal oils, vegetable oils, as well as non-metabolizable oils. Examples of vegetable oils suitable in the instant invention are corn oil, peanut oil, soybean oil, coconut oil, and olive oil. An example of animal oils is squalane. Suitable examples of non-metabolizable oils include light mineral oil, straight chained or branched saturated oils, squalane and the like.

The oil used in the adjuvant formulations disclosed herein is a light mineral oil. As used herein, the term "mineral oil" refers to a mixture of liquid hydrocarbons obtained from petrolatum via a distillation technique. The term is synonymous with "liquefied paraffin", "liquid petrolatum" and "white mineral oil." The term is also intended to include "light mineral oil," i.e., oil which is similarly obtained by distillation of petrolatum, but which has a slightly lower specific gravity than white mineral oil. See, e.g., Remington's Pharmaceutical Sciences, 18th Edition (Easton, Pa.: Mack Publishing Company, 1990, at pages 788 and 1323). Mineral oil can be obtained from various commercial sources, for example, J. T. Baker (Phillipsburg, Pa.), USB Corporation (Cleveland, Ohio). Preferred mineral oil is light mineral oil commercially available under the name DRAKEOL®.

Alternatively, the oil is SP oil, which comprises a polyoxyethylene-polyoxypropylene block copolymer (available from BASF, Mt. Olive, NJ), squalane (available from Kodak, Rochester, NY), and polyoxyethylene sorbitan monooleate (TWEEN®80, available from Sigma Chemical, St. Louis, MO). Squalane is used at approximately 5% (w/v), polyoxyethylene-polyoxypropylene block copolymer is used at approximately 2.5% (w/v), polyoxyethylene sorbitan monooleate (TWEEN®-80), is used at approximately 0.2%(w/v) prepared in a pharmaceutically acceptable diluent of either buffer, water, normal saline or cell culture growth media.

The oily phase includes oil and oil-soluble emulsifiers (e.g., SPAN 80), if any such emulsifiers are present. The volume of the oily phase is calculated as a sum of volumes of the oil and the oil soluble emulsifier. Thus, for example, if the volume of the oil is 10% and the volume of the oil-soluble emulsifier is 2% of a composition, then the oily phase would be present at 12% v/v of the composition. Similarly, if the oil is present in the amount of about 5% and the lipid-soluble emulsifier is present in the amount of about 6% of a composition, then the oily phase is present at about 11% v/v of the composition.

Emulsifiers suitable for use in the emulsions include natural biologically compatible emulsifiers and non-natural synthetic surfactants. Biologically compatible emulsifiers include phospholipid compounds or a mixture of phospholipids. Preferred phospholipids are phosphatidylcholines (lecithin), such as soy or egg lecithin. Lecithin can be obtained as a mixture of phosphatides and triglycerides by water-washing crude vegetable oils, and separating and drying the resulting hydrated gums. A refined product can be obtained by fractionating the mixture for acetone insoluble phospholipids and glycolipids remaining after removal of the triglycerides and vegetable oil by acetone washing. Alternatively, lecithin can be obtained from various commercial sources. Other suitable phospholipids include phosphatidylglycerol, phosphatidylinositol, phosphatidylserine, phosphatidic acid, cardiolipin, and phosphatidylethanolamine. The phospholipids may be isolated from natural sources or conventionally synthesized.

The emulsifiers used herein do not include lecithin, or use lecithin in an amount which is not immunologically effective.

Non-natural, synthetic emulsifiers suitable for use in the adjuvant formulations include sorbitan-based non-ionic surfactants, e.g. fatty-acid-substituted sorbitan surfactants (commercially available under the name SPAN® or ARLACEL®), fatty acid esters of polyethoxylated sorbitol (TWEEN®), polyethylene glycol esters of fatty acids from sources such as castor oil (EMULFOR®); polyethoxylated fatty acid (e.g., stearic acid available under the name SIMULSOL M-53), polyethoxylated isooctylphenol/formaldehyde polymer (TYLOXAPOL®), polyoxyethylene fatty alcohol ethers (BRIJ®); polyoxyethylene nonphenyl ethers (TRITON® N), polyoxyethylene isooctylphenyl ethers (TRITON® X). Preferred synthetic surfactants are the surfactants available under the name SPAN® and TWEEN®, such as TWEEN-80 (Polyoxyethylene (20) sorbitan monooleate) and SPAN-80 (sorbitan monooleate).

Generally speaking, the emulsifier(s) may be present in the vaccine composition in an amount of 0.01% to 10% by volume, preferably, 0.1% to 5%, more preferably 1% to 3%.

Preparation of the vaccine compositions comprising the oily phase is straightforward and entails mixing the aqueous phase comprising the antigen, the P-class immunostimulatory oligonucleotide, and, optionally, a water-soluble emulsifier, with an oily phase containing the oil and, optionally, a lipid-soluble emulsifier. The resulting mixture is emulsified.

Other components of the compositions can include pharmaceutically acceptable excipients, such as carriers, solvents, and diluents, isotonic agents, buffering agents, stabilizers, preservatives, vaso-constrictive agents, antibacterial agents, antifungal agents, and the like. Typical carriers, solvents, and diluents include water, saline, dextrose, ethanol, glycerol, oil, and the like. Representative isotonic agents include sodium chloride, dextrose, mannitol, sorbitol, lactose, and the like. Useful stabilizers include gelatin, albumin, and the like.

As used herein, "a pharmaceutically-acceptable carrier" includes any and all solvents, dispersion media, coatings, adjuvants, stabilizing agents, diluents, preservatives, antibacterial and antifungal agents, isotonic agents, adsorption delaying agents, and the like. The carrier(s) must be "acceptable" in the sense of being compatible with the other components of the compositions and not deleterious to the subject. Typically, the carriers will be sterile and pyrogen-free, and selected based on the mode of administration to be used. It is well known by those skilled in the art that the preferred formulations for the pharmaceutically acceptable carrier which comprise the compositions are those pharmaceutical carriers approved in the applicable regulations promulgated by the United States (US) Department of Agriculture or US Food and Drug Administration, or equivalent government agency in a non-US country. Therefore, the pharmaceutically accepted carrier for commercial production of the compositions is a carrier that is already approved or will be approved by the appropriate government agency in the US or foreign country.

The compositions optionally can include compatible pharmaceutically acceptable (i.e., sterile or non-toxic) liquid, semisolid, or solid diluents that serve as pharmaceutical vehicles, excipients, or media. Diluents can include water, saline, dextrose, ethanol, glycerol, and the like. Isotonic agents can include sodium chloride, dextrose, mannitol, sorbitol, and lactose, among others. Stabilizers include albumin, among others.

The compositions can also contain antibiotics or preservatives, including, for example, gentamicin, merthiolate, or chlorocresol. The various classes of antibiotics or preservatives from which to select are well known to the skilled artisan.

### Administration and Use of the Compositions

Dose sizes of the compositions typically range from about 0.5 mL to about 5 mL, inclusive, depending on the subject and the antigen. For example, for a canine or feline, a dose of about 0.5 to about 1 mL is typically used, while in larger animals a dose of about 1-5 mL is typically used. However, these adjuvants also can be formulated in microdoses, wherein doses of about 100 to about 500 µL can be used.

The routes of administration for the adjuvant compositions include, without limitations, subcutaneous, intramuscular, parenteral, oral, oronasal, intranasal, intratracheal, topical, etc. Any suitable device may be used to administer the compositions, including syringes, droppers, needleless injection devices, patches, and the like. The route and device selected for use will depend on the composition of the adjuvant, the antigen, and the subject, and such are well known to the skilled artisan.

The invention will be further described in the following examples.

### EXAMPLES

### Example 1: Leishmania antigen

Fifty laboratory-reared male and female beagle dogs were obtained from Marshall BioResources (NY), and were randomized 5 dogs per each of 10 groups (Table 1). The dogs were between 3 and 4 months of age at the time of the first injection. Three injections were administered at 21-day intervals; injection sites were observed, and blood samples were collected at prescribed intervals for assay. P-CpG of SEQ ID NO: 8 was used for this study.

**Table 1: Experimental Design for Leishmania Antigen Study in Dogs**

| **Group** | **Antigen** | **Adjuvant** | | **No. Doses** | **Route & Vol/Dose** |
|---|---|---|---|---|---|
| (N=5) | | **1** | **2** | Days | |
| T01 | FML | MDP (200µg) | | 3 doses Days: **0,21 & 42** | **SC** 1 ml |
| T02 | Leishmune® | Per marketed formulation | | | |
| T03 | FML | P-CpG (100µg) | SP Oil (10%) | | |
| T04 | FML | P-CpG (200µg) | SP Oil (10%) | | |
| T05 | FML | P-CpG (100µg) | QC (50µg / 50µg) | | |
| T06 | FML | P-CpG (200µg) | QC(50µg / 50 µg) | | |
| T07 | FML | E-SA (5% v/v) | SP-Oil (10%) | | |
| T08 | FML | MDP (200 µg) | QC(50µg / 50 µg) | | |
| T09 | FML | PBS | --- | | |
| T10 | PBS | --- | --- | | |

| | | | | | |
|---|---|---|---|---|---|
| CpG - P class oligonucleotide Q - Quil A C - Cholesterol MDP - Muramyl Dipeptide FML - Fucose Mannose Ligand E-SA - Emulsigen®-SA | | | | | |

PMBC were isolated on days -1, 7, 28, and 49. Isolated PBMC were cultured in (MEDIA) and stimulated with FML (20 ug/ml), LICE (*Leishmania Infantum* Crude Extract, 17.5 ug/ml) and ConA (Concanavalin A, 2.5 ug/ml). IFN-g was measured by ELISpot using commercially available kits. The results of the experiments are summarized in the tables below.

**Table 2: PBMC ELISPot Stimulation Index Results for Leishmania Antigen Study**

| Group | **FML Ag - Stimulation Index on Day:** | | | | **LICE Ag stimulation Index on Day:** | | | |
|---|---|---|---|---|---|---|---|---|
| | **-1** | **7** | **28** | **49** | **-1** | **7** | **28** | **49** |
| T01 | 2 | 5 | 3 | 8 | 1 | 1 | 1 | 3 |
| T02 | 2 | 6 | 6 | 3 | 1 | 2 | 2 | 2 |
| T03 | 2 | 4 | 4 | 3 | 1 | 2 | 1 | 1 |
| T04 | 2 | 4 | 4 | 3 | 1 | 1 | 1 | 1 |
| T05 | 2 | 9 | 25 | 28 | 1 | 3 | 5 | 12 |
| T06 | 2 | 7 | 15 | 21 | 1 | 2 | 4 | 6 |
| T07 | 2 | 5 | 13 | 11 | 1 | 2 | 2 | 3 |
| T08 | 2 | 6 | 18 | 18 | 1 | 2 | 5 | 6 |
| T09 | 2 | 3 | 3 | 4 | 1 | 2 | 2 | 1 |
| T10 | 3 | 3 | 3 | 3 | 2 | 1 | 2 | 2 |

**Table 3: PBMC ELISPot Spot Forming Cells (SFC)/ 10⁶ PBMCs Results for Leishmania Antigen Study**

| Group | **FML Ag - SFCs/10⁶ PBMCs on Day:** | | | | **LICE Ag SFCs/10⁶ PBMCs on Day:** | | | |
|---|---|---|---|---|---|---|---|---|
| | **-1** | **7** | **28** | **49** | **-1** | **7** | **28** | **49** |
| T01 | 20 | 25 | 29 | 64 | 6 | 4 | 2 | 13 |
| T02 | 12 | 69 | 306 | 374 | 3 | 12 | 50 | 94 |
| T03 | 5 | 230 | 897 | 1165 | 3 | 22 | 72 | 91 |
| T04 | 16 | 304 | 519 | 1054 | 1 | 50 | 58 | 159 |
| T05 | 14 | 115 | 638 | 977 | 6 | 31 | 132 | 403 |
| T06 | 10 | 107 | 570 | 766 | 0 | 28 | 113 | 193 |
| T07 | 16 | 28 | 85 | 122 | 2 | 5 | 7 | 21 |
| T08 | 10 | 64 | 295 | 326 | 1 | 17 | 56 | 107 |
| T09 | 21 | 29 | 74 | 97 | 4 | 4 | 8 | 10 |
| T10 | 29 | 16 | 25 | 22 | 8 | 2 | 4 | 8 |

Taken together, these data demonstrate that CpG (100 µg) with QC appears to be the best adjuvant for FML, with respect to IFN-g Stimulation Index. CpG (100 µg) with SP oil induced the highest IFN-g SFCs, but also had the highest background stimulation. Surprisingly, the positive control, Leishmune vaccine was slightly better than the Neg. Control (PBS or FML alone), even though it was adjuvanted with 500 µg Quil A per dose. In other words, the addition of CpG allowed to reduce Quil A 10 times and still get superior results. The effects of previously known adjuvants MPD and E-SA were comparable or slightly better than Leishmune®, but inferior to CpG with QC.

## Claims

1. A vaccine composition comprising an antigen component and an adjuvant component, wherein the adjuvant component comprises a P-class CpG oligonucleotide and a combination of a saponin and a sterol; and wherein said antigen component is a Leishmania antigen.

2. A vaccine composition of claim 1, wherein the adjuvant consists essentially of the P-class CpG oligonucleotide and the combination of the saponin and the sterol.

3. The vaccine composition of claim 1 or 2, which is a non-liposomal composition.

4. The vaccine composition of any one of claims 1-3, wherein the saponin is Quil A and the sterol is cholesterol.

5. The vaccine composition of any one of claims 1-4, wherein the saponin is present in the amount of 5-100 µg/ml.

6. The vaccine composition of any one of claims 1-5, wherein the sterol is present in the amount of 5-100 µg/ml.

7. The vaccine composition of any one of claims 1-6, wherein the sterol and the saponin each are present in the amount of 50 µg/ml.

8. The vaccine composition of any one of the preceding claim, wherein the antigen is Fucose Mannose Ligand (FML).

9. The vaccine composition of any one of claims 1-8, wherein said P-class CpG oligonucleotide is present in the amount of 20-500 µg /ml.

10. The vaccine composition of claim 9, wherein said P-class CpG oligonucleotide is present in the amount of 100-250 µg/ml.

11. The vaccine composition of any one of claims 1-10, wherein said P-class CpG oligonucleotide is modified.

12. The vaccine composition of claim 11, wherein said modified P-class CpG oligonucleotide is JU-modified or E-modified.

13. The vaccine composition of claim 12, wherein said modified P-class CpG oligonucleotide is SEQ ID NO: 7, SEQ ID NO: 8 or SEQ ID NO: 9.

## Patentansprüche

1. Impfstoffzusammensetzung, umfassend eine Antigenkomponente und eine Adjuvanskomponente, wobei die Adjuvanskomponente ein P-Klasse-CpG-Oligonukleotid und eine Kombination eines Saponins und eines Sterols umfasst, und wobei die Antigenkomponente ein Leishmania-Antigen ist.

2. Impfstoffzusammensetzung gemäß Anspruch 1, wobei das Adjuvans im Wesentlichen aus dem P-Klasse-CpG-Oligonukleotid und der Kombination des Saponins und des Sterols besteht.

3. Impfstoffzusammensetzung gemäß Anspruch 1 oder 2, welche eine nicht-liposomale Zusammensetzung ist.

4. Impfstoffzusammensetzung gemäß einem der Ansprüche 1 bis 3, wobei das Saponin Quil-A und das Sterol Cholesterin ist.

5. Impfstoffzusammensetzung gemäß einem der Ansprüche 1 bis 4, wobei das Saponin in einer Menge von 5 bis 100 µg/ml anwesend ist.

6. Impfstoffzusammensetzung gemäß einem der Ansprüche 1 bis 5, wobei das Sterol in einer Menge von 5 bis 100 µg/ml anwesend ist.

7. Impfstoffzusammensetzung gemäß einem der Ansprüche 1 bis 6, wobei das Sterol und das Saponin jeweils in einer Menge von 50 µg/ml anwesend ist.

8. Impfstoffzusammensetzung gemäß einem der vorhergehenden Ansprüche, wobei das Antigen Fucose-Mannose-Ligand (FML) ist.

9. Impfstoffzusammensetzung gemäß einem der Ansprüche 1-8, wobei das P-Klasse-CpG-Oligonukleotid in einer Menge von 20 bis 500µg/ml anwesend ist.

10. Impfstoffzusammensetzung gemäß Anspruch 9, wobei das P-Klasse-CpG-Oligonukleotid in einer Menge von 100 bis 250 µg/ml anwesend ist.

11. Impfstoffzusammensetzung gemäß einem der Ansprüche 1 bis 10, wobei das P-Klasse-CpG-Oligonukleotid modifiziert ist.

12. Impfstoffzusammensetzung gemäß Anspruch 11, wobei das modifizierte P-Klasse-CpG-Oligonukleotid JU-modifiziert oder E-modifiziert ist.

13. Impfstoffzusammensetzung gemäß Anspruch 12, wobei das modifizierte P-Klasse-CpG-Oligonukleotid SEQ ID NO: 7, SEQ ID NO: 8 oder SEQ ID NO:9 ist.

## Revendications

1. Composition vaccinale comprenant un composant antigène et un composant adjuvant, dans laquelle le composant adjuvant comprend un oligonucléotide CpG de classe P et une combinaison d'une saponine et d'un stérol ; et dans laquelle ledit composant antigène est un antigène de Leishmania.

2. Composition vaccinale selon la revendication 1, dans laquelle l'adjuvant est constitué essentiellement de l'oligonucléotide CpG de classe P et de la combinaison de la saponine et du stérol.

3. Composition vaccinale selon la revendication 1 ou 2, qui est une composition non liposomique.

4. Composition vaccinale selon l'une quelconque des revendications 1 à 3, dans laquelle la saponine est Quil A et le stérol est le cholestérol.

5. Composition vaccinale selon l'une quelconque des revendications 1 à 4, dans laquelle la saponine est présente dans la quantité de 5 à 100 µg/ml.

6. Composition vaccinale selon l'une quelconque des revendications 1 à 5, dans laquelle le stérol est présent dans la quantité de 5 à 100 µg/ml.

7. Composition vaccinale selon l'une quelconque des revendications 1 à 6, dans laquelle le stérol et la saponine sont chacun présents dans la quantité de 50 µg/ml.

8. Composition vaccinale selon l'une quelconque des revendications précédentes, dans laquelle l'antigène est le ligand fucose-mannose (FML).

9. Composition vaccinale selon l'une quelconque des revendications 1 à 8, dans laquelle ledit oligonucléotide CpG de classe P est présent dans la quantité de 20 à 500 µg/ml.

10. Composition vaccinale selon la revendication 9, dans laquelle ledit oligonucléotide CpG de classe P est présent dans la quantité de 100 à 250 µg/ml.

11. Composition vaccinale selon l'une quelconque des revendications 1 à 10, dans laquelle ledit oligonucléotide CpG de classe P est modifié.

12. Composition vaccinale selon la revendication 11, dans laquelle ledit oligonucléotide CpG de classe P modifié est JU-modifié ou E-modifié.

13. Composition vaccinale selon la revendication 12, dans laquelle ledit oligonucléotide CpG de classe P est SEQ ID NO : 7, SEQ ID NO : 8 ou SEQ ID NO : 9.
